# EUROPÄISCHE PATENTSCHRIFT

(11) **EP 1 086 667 B1**
(45) Veröffentlichungstag und Bekanntmachung des Hinweises auf die Patenterteilung: **04.08.2004**
(21) Anmeldenummer: 00810764.1
(22) Anmeldetag: 28.08.2000
(51) Int. Cl.: A61F 2/38

(54) **Tibiateil für eine Kniegelenkprothese und Bausatz mit einem solchen Tibiateil**
Tibial element for a knee prothesis and modular set comprising this tibial element
Pièce tibiale pour prothèse du genou et ensemble modulaire la comprenant

(30) Priorität: 24.09.1999 EP 99810862
(43) Veröffentlichungstag der Anmeldung: 28.03.2001
(73) Patentinhaber: Centerpulse Orthopedics Ltd., 6340 Baar (CH)
(72) Erfinder: Brack, René, 6330 Cham (CH); Scherrer, Roger, 8207 Schaffhausen (CH); Leclercq, Vincent, 8400 Winterthur (CH)
(74) Vertreter: Manitz, Finsterwald & Partner Gbr

(56) Entgegenhaltungen:
- EP-A- 0 519 873
- EP-A- 0 904 749
- EP-A- 0 913 132

## Beschreibung

Die Erfindung betrifft ein Tibiateil für eine Kniegelenkprothese und einen Bausatz zum Zusammenstellen einer Kniegelenkprothese gemäss dem Oberbegriff des jeweiligen unabhängigen Anspruchs.

Kniegelenkprothesen umfassen üblicherweise ein Tibiateil, das in der Tibia fixiert wird, ein Femurteil, das im Femur fixiert wird, sowie einen dazwischen angeordneten Lagerkörper, der oft auch als Meniskusteil bezeichnet wird. Das Tibiateil weist eine Tibialagerfläche zum Stützen des Lagerkörpers auf. Der Lagerkörper ist auf seiner der Tibialagerfläche abgewandten Seite - also auf der dem Femur zugewandten Seite - mit Lagerschalen versehen, welche mit den Kondylen (Laufflächen) des Femurteils zusammenwirken.

Heutzutage stehen Kniegelenkprothesen in einer grossen Anzahl verschiedener Prothesentypen zur Verfügung, wobei sich ihre Funktionsprinzipien oft stark voneinander unterscheiden. Dies hängt unter anderem damit zusammen, dass die Prothese je nach Zustand der Bänder (Kreuzbänder, Seitenbänder) bei dem jeweiligen Patienten die verschiedenen Funktionen der Bänder ganz oder teilweise übernehmen muss.

So sind beispielsweise Prothesentypen bekannt (siehe z.B. EP-A-0,923,916), bei denen das Meniskusteil auf der Tibialagerfläche des Tibiateils fixiert ist, sodass das Meniskusteil relativ zum Tibiateil unbeweglich angeordnet ist.

Weiterhin sind Prothesentypen bekannt, bei denen das Meniskusteil relativ zum Tibiateil bewegbar auf der Tibialagerfläche gelagert ist, d.h. also entweder auf der Tibialagerfläche gleitend verschiebbar oder auf der Tibialagerfläche drehbar ist oder beides.

In der EP-A-0,913,132 wird beispielsweise eine Kniegelenkprothese gezeigt, bei welcher das Meniskusteil zwar gleitend verschiebbar, aber im wesentlichen drehfest auf der Tibialagerfläche des Tibiateils gelagert ist, sodass das Meniskusteil relativ zum Tibiateil auf der Tibialagerfläche zwar in anteriorer/posteriorer Richtung verschiebbar, aber im wesentlichen nicht drehbar ist.

Ferner sind Prothesentypen bekannt, bei denen das Meniskusteil relativ zur Tibialagerfläche zwar drehbar, aber nicht verschiebbar auf dem Tibiateil gelagert ist, sodass das Meniskusteil relativ zum Tibiateil nur rotierbar ist, aber nicht verschoben werden kann.

Schliesslich sind z.B. aus der EP-A-0,519,873 Prothesentypen bekannt, bei denen das Meniskusteil relativ zur Tibialagerfläche sowohl gleitend verschiebbar als auch drehbar ist.

Darüberhinaus sind Prothesentypen bekannt, bei denen Mittel vorgesehen sind, um das Femurteil bei der Flexion (Beugung) in der posterioren Stellung relativ zum Meniskusteil bzw. relativ zum Tibiateil zu stabilisieren. Dieser Prothesentyp findet insbesondere dann Verwendung, wenn das hintere Kreuzband nicht mehr vorhanden oder nicht mehr funktionstüchtig ist.

Insbesondere bei Fällen, bei denen die Seitenbänder nicht mehr vorhanden oder nicht mehr ausreichend funktionstüchtig sind, werden Prothesentypen verwendet, bei denen Mittel zur seitlichen Stabilisierung (VarusNalgus-Stabilisierung) vorgesehen sind.

Schon aus dieser nicht abschliessenden Aufzählung wird ersichtlich, dass eine Vielzahl von unterschiedlichen Ausführungsformen der einzelnen Bauteile einer Kniegelenkprothese, insbesondere auch eine Vielzahl unterschiedlicher Tibiateile, bereitgestellt werden muss, damit für den jeweiligen Patienten die jeweils optimale Prothese zusammengestellt werden kann.

Für jeden Prothesentyp existiert ein speziell an diesen Typ angepasstes Tibiateil, um ein möglichst gutes Zusammenwirken mit den übrigen Bestandteilen der Prothese zu gewährleisten. Das soll heissen: Ein Tibiateil, das für einen bestimmten Prothesentyp hergestellt wurde, lässt sich in der Regel nicht für andere Prothesentypen verwenden. Dadurch bedingt existieren zum Teil erhebliche konstruktive Unterschiede zwischen Tibiateilen, die zu unterschiedlichen Prothesentypen gehören.

Dies ist jedoch insbesondere unter herstellungstechnischen Aspekten ein Nachteil, denn für jeden Prothesentyp muss das Tibiateil gemäss einem für das jeweilige Tibiateil spezifischen Herstellungsprozess produziert werden. Um also mehrere Tibiateile für unterschiedliche Prothesentypen bereitzustellen, müssen auch mehrere, teilweise sehr unterschiedliche Herstellungsprozesse angewandt werden, was von der technischen Seite her sehr aufwendig und ausserdem auch ökonomisch unvorteilhaft ist.

Will man einen Bausatz schaffen, mit dem verschiedene Prothesentypen zusammengestellt werden können, so muss dieser Bausatz eine Vielzahl von Tibiateilen umfassen, die sich bezüglich ihrer funktionellen Ausgestaltung voneinander unterscheiden. Da zusätzlich für jeden Prothesentyp auch noch mehrere Tibiateile unterschiedlicher Grösse in dem Bausatz vorhanden sein müssen, um den unterschiedlichen anatomischen Gegebenheiten der Patienten Rechnung zu tragen, umfasst ein solcher Bausatz zwangsläufig eine sehr grosse Anzahl einzelner Bauteile.

Hier will die Erfindung Abhilfe schaffen. Es ist folglich eine Aufgabe der Erfindung, ein Tibiateil vorzuschlagen, welches die Herstellung für unterschiedliche Prothesentypen erheblich vereinfacht. Ferner soll durch das Tibiateil ein Bausatz zum Zusammenstellen einer Kniegelenkprothese ermöglicht werden, der erheblich weniger verschiedene Tibiateile umfasst, ohne dass dafür Zugeständnisse vonnöten sind an die Flexibilität bezüglich unterschiedlicher Prothesentypen, die mit dem Bausatz realisierbar sind, oder Zugeständnisse an die Funktionalität der Kniegelenkprothese.

Die diese Aufgaben lösenden Gegenstände der Erfindung sind durch die Merkmale der unabhängigen Ansprüche gekennzeichnet.

Erfindungsgemäss wird also ein Tibiateil für eine Kniegelenkprothese vorgeschlagen mit einer Tibialagerfläche zum Stützen eines Lagerkörpers (Meniskusteils), und mit einer Bohrung zur Aufnahme eines Führungselements, wobei in der Tibialagerfläche eine Aussparung für das hintere Kreuzband vorgesehen ist, und wobei die Bohrung derart ausgestaltet ist, dass sie je nach Ausgestaltung des Führungselements dieses entweder drehfest bezüglich des Tibiateils oder drehbar bezüglich des Tibiateils aufnehmen kann.

Diese Massnahmen ermöglichen ein Tibiateil, das für mehrere, funktionell verschiedene Prothesentypen geeignet ist, das heisst, das gleiche Tibiateil kann mit verschiedenen Lagerkörpern und/oder Femurteilen und/oder Führungselementen kombiniert werden, um so verschiedene Prothesentypen zusammenstellen zu können. Durch die Ausnehmung zur Aufnahme des hinterer Kreuzbands ist das Tibiateil für solche Prothesentypen geeignet, bei denen das hintere Kreuzband erhalten bleibt und funktionstüchtig ist. Durch die Ausgestaltung der Bohrung in der Tibialagerfläche eignet sich das erfindungsgemässe Tibiateil sowohl für Prothesentypen, bei denen der Lagerkörper relativ zum Tibiateil drehfest gelagert ist, als auch für Prothesentypen, bei denen der Lagerkörper drehbar auf der Tibialagerfläche gelagert ist.

Die Variabilität des erfindungsgemässen Tibiateils bzw. seine Kompatibilität mit verschiedenen Prothesentypen bedeutet eine erhebliche Vereinfachung des Herstellungsprozesses, weil für verschiedenen Prothesentypen das gleiche Tibiateil eingesetzt werden kann.

Vorzugsweise ist das Tibiateil mit einem Ansatzstück versehen, welches der Befestigung des Tibiateils in der Tibia dient, wobei Mittel vorgesehen sind, um das Ansatzstück fest mit einem separaten Verankerungsschaft zu verbinden, falls ein separater Verankerungsschaft aufgrund anatomischer Gegebenheiten angezeigt erscheint. Hierdurch ist es möglich, das Tibiateil mit unterschiedlich ausgestalteten, insbesondere unterschiedlich langen Verankerungsschäften (sofern angezeigt) zu versehen, um so die Verankerung des Tibiateils in der Tibia jeweils optimal auf den speziellen Anwendungsfall abstimmen zu können.

Gemäss einem ersten Ausführungsbeispiel sind am Tibiateil Befestigungsmittel vorgesehen, um den Lagerkörper auf der Tibialagerfläche zu fixieren. Dieses Ausführungsbeispiel eignet sich insbesondere für solche Prothesentypen, bei denen der Lagerkörper relativ zum Tibiateil unbeweglich ist.

In einem zweiten Ausführungsbeispiel sind diese Befestigungsmittel nicht vorgesehen, sodass der Lagerkörper relativ zum Tibiateil beweglich auf der Tibialagerfläche gelagert ist.

Im Hinblick auf einen möglichst einfachen Herstellungsprozess ist es vorteilhaft, wenn sich diese beiden Ausführungsbeispiele nur durch die Befestigungsmittel unterscheiden und ansonsten praktisch baugleich sind.

Gemäss einem weiteren Aspekt der Erfindung wird ein Bausatz zum Zusammenstellen einer Kniegelenkprothese vorgeschlagen. Der Bausatz umfasst mindestens ein Tibiateil, das eine Tibialagerfläche aufweist, ferner mindestens einen Lagerkörper, der auf der Tibialagerfläche lagerbar ist und der auf seiner der Tibialagerfläche abgewandten Seite Lagerschalen aufweist, sowie mindestens ein Femurteil, welches Kondylen zum Zusammenwirken mit den Lagerschalen des Lagerkörpers aufweist. Das Tibiateil dieses Bausatzes ist dabei als erfindungsgemässes Tibiateil ausgebildet, wie es oben bereits geschildert ist.

Da das erfindungsgemässe Tibiateil für mehrere, funktionell verschiedene Prothesentypen geeignet ist, ist es nicht notwendig, in einem solchen Bausatz für jeden Prothesentyp ein speziell an diesen Prothesentyp angepasstes Tibiteil vorzusehen. Vielmehr kann das gleiche Tibiateil mit funktionell verschiedenen bzw. funktionell verschiedenartig zusammenwirkenden Komponenten (z. B. Lagerkörpern, Femurteilen, Führungselementen) kombiniert werden, ohne dass dafür Zugeständnisse an die Funktionstüchtigkeit der Kniegelenkprothese notwendig sind. Dadurch werden für den Bausatz erheblich weniger verschiedene Tibiateile benötigt, ohne dass sich dadurch die Flexibilität des Bausatzes bezüglich der zusammenstellbaren Prothesentypen vermindert. Dies bedeutet nicht nur in Bezug auf die Anzahl der in einem Bausatz enthaltenen Tibiateile, sondern auch herstellungstechnisch eine deutliche Vereinfachung.

Vorzugsweise sind in dem Bausatz mindestens ein Tibiateil und mindestens ein Lagerkörper so ausgebildet, dass der Lagerkörper auf der Tibialagerfläche fixierbar ist, damit auch Prothesentypen mit relativ zum Tibiateil fixiertem Lagerkörper realisierbar sind.

Um Prothesentypen mit relativ zum Tibiateil sowohl verschiebbarem als auch drehbaren Lagerkörper zu realisieren, umfasst der Bausatz bevorzugt mindestens einen Lagerkörper, der gleitend verschiebbar auf der Tibialagerfläche lagerbar ist, und mindestens ein Führungselement, das als Lenker ausgebildet ist, dessen eines Ende drehbar in der Bohrung des Tibiateils lagerbar ist, und der einen Führungsabschnitt aufweist, welcher so ausgebildet ist und mit dem Lagerkörper zusammenwirkt, dass der Lagerkörper relativ zur Tibialagerfläche in anteriorer/posteriorer Richtung verschiebbar ist.

Für Prothesentypen mit einem drehbar - aber nicht verschiebbar - gelagerten Lagerkörper ist in dem Bausatz vorzugsweise mindestens ein Lagerkörper vorgesehen, welcher auf seiner der Tibialagerfläche zugewandten Seite ein angeformtes oder fixiertes Führungselement aufweist, mittels welchem der Lagerkörper drehbar in der Bohrung des Tibiateils lagerbar ist.

Für Prothesentypen mit posteriorer Stabilisierung umfasst der Bausatz bevorzugt mindestens einen Lagerkörper, der ein axial durchgängiges Langloch aufweist, sowie mindestens ein Führungselement, welches sich durch das Langloch des Lagerkörpers hindurch erstreckt, wobei das eine Ende des Führungselements als Zapfen ausgebildet ist, der sich in die Bohrung im Tibiateil hinein erstreckt und dort drehbar gelagert ist, und das andere Ende zwischen die Kondylen des Femurteils hineinragt und eine Führungsfläche zum Zusammenwirken mit einem im Femurteil vorgesehenen Stabilisierungselement aufweist.

Ferner ist es vorteilhaft für Fälle, in denen das hintere Kreuzband und die Seitenbänder nicht mehr existieren oder nicht mehr funktionstüchtig sind, wenn der Bausatz mindestens ein Führungselement mit einem Führungsstück und einem Kopplungsstück aufweist, wobei das Führungsstück so ausgebildet ist, dass es sowohl drehfest bezüglich des Tibiateils in der Bohrung des Tibiateils angeordnet werden kann, als auch drehfest mit dem Lagerkörper verbindbar ist. Das Kopplungsstück ist drehbar in dem Führungsstück gelagert und weist ein Stabilisierungsstück auf, welches zwischen die Kondylen des Femurteils reicht, um dort mit einem Stabilisierungselement zusammenzuwirken. Hiermit lassen sich posterior stabilisierte Prothesentypen mit zusätzlicher Varus/Valgus-Stabilisierung (in Ermangelung funktionstüchtiger Seitenbänder) realisieren.

Das Stabilisierungselement am Femurteil umfasst bei einer vorteilhaften Ausführungsvariante einen wandartig ausgebildeten Verbindungssteg, der zusammen mit zwei Seitenwänden einen Kasten bildet, welcher zwischen den beiden Kondylen des Femurteils angeordnet ist und welcher mit der Führungsfläche des Führungselements bzw. mit dem Stabilisierungsstück des Kopplungsstücks des Führungselements zusammenwirkt. Je nachdem, mit welchem Typ Kniegelenkprothese der wandartig ausgebildete Verbindungssteg zusammenwirkt, gelangt bei der Flexion entweder die Aussenwand des wandartigen Verbindungsstegs in Kontakt mit der Führungsfläche des Führungselements, oder aber die Innenwand des wandartigen Verbindungsstegs gelangt in Kontakt mit der Führungsfläche des Kopplungsstücks des Führungselements. Darüberhinaus erfolgt bei der letztgenannten Variante auch noch eine Varus/Valgus-Stabilisierung mit Hilfe des Stabilisierungsstücks, welches zwischen die Kondylen des Femurteils reicht und dessen Seitenflächen (Stabilisierungsflächen) mit den Seitenwänden des Kastens zusammenwirken.

In einem bevorzugten Ausführungsbeispiel umfasst der erfindungsgemässe Bausatz alle die vorstehend aufgezählten Elemente. Ein solcher Bausatz zeichnet sich durch seine extrem hohe Flexibilität aus, das heisst, abhängig von der jeweiligen Indikation können die verschiedenartigen Prothesentypen in modularer Weise aus diesem Bausatz zusammengestellt werden. Ein besonderer Vorteil ist dabei, dass diese Flexibilität auch noch intraoperativ gegeben ist, das heisst, dass der Orthopäde bei Fällen, bei denen er trotz sorgfältiger präoperativer Planung bei der Operation besondere anatomische Verhältnisse vorfindet, noch während der Operation denjenigen Prothesentyp auswählen und aus dem Bausatz zusammenstellen kann, welcher für den jeweiligen Anwendungsfall am besten geeignet ist. Diese hohe Flexibilität ist insbesondere deshalb von Vorteil, weil z.B. der Zustand der Bänder trotz sorgfältiger präoperativer Planung nicht immer mit absoluter Sicherheit festgestellt werden kann oder auch das Knochenmaterial tatsächlich in einem besseren oder schlechteren Zustand ist, als es die präoperative Analyse zeigt, sodass ein anderen Prothesentyp als der ursprünglich präoperativ geplante Prothesentyp eher angezeigt erscheint.

Da das erfindungsgemässe Tibiateil in modularer Weise mit den anderen Komponenten des Bausatzes zu einer Mehrzahl funktionell verschiedener Prothesentypen kombinierbar ist, kann die Anzahl der Tibiateile in dem Bausatz vergleichsweise gering gehalten werden. In dem erwähnten bevorzugten Ausführungsbeispiel des Bausatzes sind vorzugsweise höchstens vier verschiedenartige Tibiateile vorhanden, nämlich zwei für zementierte und zwei für zementfreie Kniegelenkprothesen, wobei in beiden Fällen - zementiert und zementfrei - jeweils ein Tibiateil für fixierte Lagerkörper und eines für mobile Lagerkörper vorgesehen ist. Die für zementierte Prothesen einerseits und zementfreie Prothesen andererseits vorgesehenen Tibiateile unterscheiden sich von ihrer geometrischen Ausgestaltung nicht. Die Tibiateile für zementierte Prothesen weisen an ihrer der Tibia zugewandten Unterseite Zementtaschen auf. Bei den Tibiateilen für zementfreie Prothesen sind keine Zementtaschen vorgesehen, sondern stattdessen ist an der der Tibia zugewandten Unterseite das Tibiateil mit einer Materialschicht versehen, welche das Einwachsen des Knochens fördert, beispielsweise mit porösem Titan. Dies kann herstellungstechnisch beispielsweise so erfolgen, dass die Tibiateile für zementfreie Anwendungen aus Tibiateilen für zementierte Anwendungen so gewonnen werden, dass die Zementtaschen mit porösem Titan gefüllt werden.

Der Orthopäde kann nach dem Auswählen des geeigneten Prothesentyps, Resektion der Tibia nach einer vorgegebenen Schnittführung, die für alle Tibiateile gleich ist, ein Tibiateil auswählen, dieses an der Tibia fixieren, anschliessend ein entsprechendes Femurteil auswählen, dieses am Femur fixieren, und schliesslich einen geeigneten Lagerkörper auswählen und einsetzen. Sollte sich dabei während der Operation herausstellen, dass aufgrund der anatomischen Verhältnisse ein anderer Prothesentyp eher angezeigt sein sollte, so kann der Orthopäde sich intraoperativ noch für einen anderen Typ von Kniegelenkprothese entscheiden. Bei Tibiateilen für fixierte Lagerkörper hat er z.B. noch die Wahl zwischen einem Lagerkörper mit normaler oder besonders hoher Kongruenz, bei Tibiateilen für gleitend verschiebbar gelagerte und/oder drehbar gelagerte Lagerkörper hat er noch die Wahl zwischen einem ganzen Spektrum von Prothesentypen.

Der Orthopäde kann also zunächst entscheiden, ob aufgrund der anatomischen Verhältnisse der gleiche Prothesentyp beibehalten wird oder ein anderer Prothesentyp zur Anwendung gelangen soll. Letzteres ist bei einer Revision klarerweise der häufigere Fall. Sodann kann der Orthopäde entscheiden, ob für den neuen Prothesentyp das ursprüngliche Femurteil und/oder das ursprüngliche Tibiateil beibehalten werden kann, was unter anderem von dem Prothesentyp abhängig ist, für den sich der Orthopäde bei der Revision entscheidet. Danach kann der Orthopäde einen für den gewählten Prothesentyp geeigneten Lagerkörper aus dem erfindungsgemässen Bausatz auswählen und dann das neue Tibiateil oder das neue Femurteil oder aber - bei Beibehaltung von Tibiateil und Femurteil - nur den neuen Lagerkörper einsetzen.

Im Folgenden wird die Erfindung sowohl in apparativer als auch in verfahrenstechnischer Hinsicht anhand der Zeichnung und anhand von

Ausführungsbeispielen näher erläutert. In der schematischen Zeichnung zeigen:
- Fig. 1: eine Aufsicht auf ein erstes Ausführungsbeispiel des erfindungsgemässen Tibiateils,
- Fig. 2: einen perspektivischen Längsschnitt durch das erste Ausführungsbeispiel des erfindungsgemässen Tibiateils,
- Fig. 3: eine Ansicht der Unterseite des ersten Ausführungsbeispiels des erfindungsgemässen Tibiateils,
- Fig. 4: eine Aufsicht auf ein zweites Ausführungsbeispiel des erfindungsgemässen Tibiateils,
- Fig. 5: einen perspektivischen Längsschnitt durch das zweite Ausführungsbeispiel des erfindungsgemässen Tibiateils,
- Fig. 6: ein erster Typ (Typ "CR") einer Kniegelenkprothese mit einem erfindungsgemässen Tibiateil,
- Fig. 7: ein zweiter Typ (Typ "UCOR") einer Kniegelenkprothese mit einem erfindungsgemässen Tibiateil,
- Fig. 8: ein dritter Typ (Typ "PS") einer Kniegelenkprothese mit einem erfindungsgemässen Tibiateil,
- Fig. 9: ein vierter Typ (Typ "SC") einer Kniegelenkprothese mit einem erfindungsgemässen Tibiateil,
- Fig. 10: den dritten Typ (Typ "PS") der Kniegelenkprothese im Zusammenbau in perspektivischer Darstellung, im Zustand der Extension
- Fig. 11: die Kniegelenkprothese gemäss Fig. 10 im Zustand der Extension, im Längsschnitt und in Seitenansicht,
- Fig. 12: die Kniegelenkprothese gemäss Fig. 10 im Zustand der Flexion, im Längsschnitt und in Seitenansicht,
- Fig. 13: die Kniegelenkprothese gemäss Fig. 10 im Zustand der maximalen Flexion, im Längsschnitt und in Seitenansicht,
- Fig. 14: den vierten Typ (Typ "SC") der Kniegelenkprothese im Zusammenbau in perspektivischer Darstellung, im Zustand der Extension
- Fig. 15: die Kniegelenkprothese gemäss Fig. 14 im Zustand der Extension, im Längsschnitt und in Seitenansicht,
- Fig. 16: die Kniegelenkprothese gemäss Fig. 14 im Zustand der Flexion, im Längsschnitt und in Seitenansicht,
- Fig. 17: die Kniegelenkprothese gemäss Fig. 14 im Zustand der maximalen Flexion, im Längsschnitt und in Seitenansicht
und
- Fig. 18: eine Übersicht über Kombinationsmöglichkeiten eines Bausatzes, der erfindungsgemässe Tibiateile umfasst.

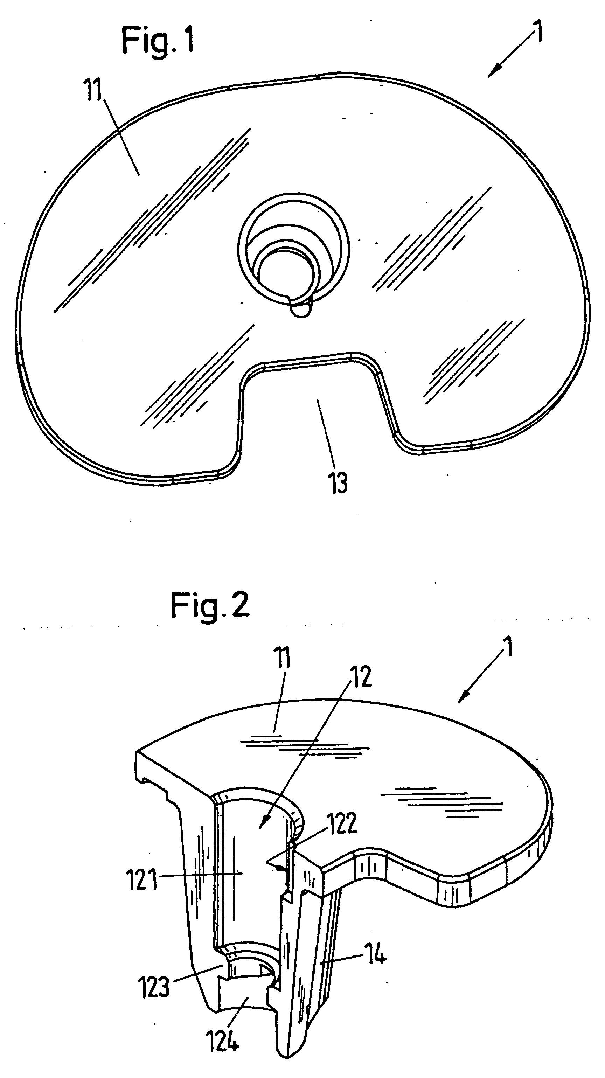

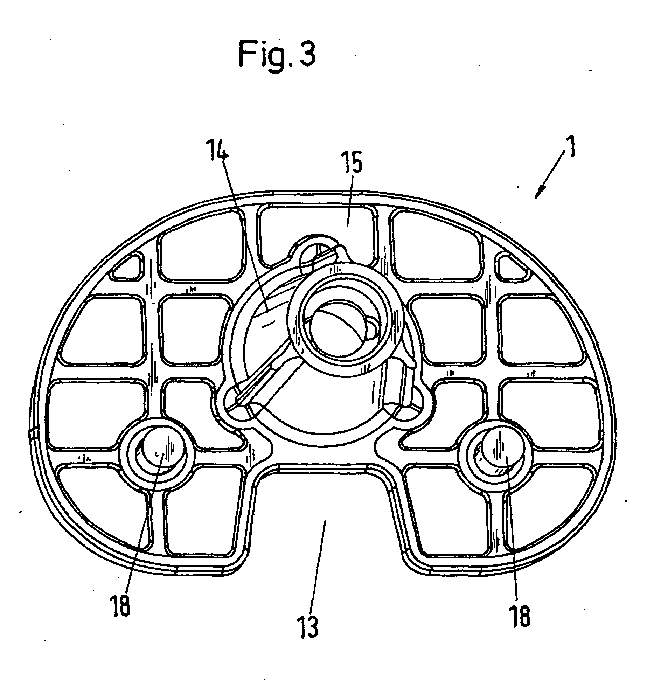

In den Figuren 1-3 ist ein erstes Ausführungsbeispiel eines erfindungsgemässen Tibiateils für eine Kniegelenkprothese dargestellt, das gesamthaft mit dem Bezugszeichen 1 bezeichnet ist. Fig. 1 zeigt das Tibiateil 1 in einer Aufsicht, in Fig. 2 ist ein perspektivischer Längsschnitt entlang der Längsachse des Tibiateils 1 dargestellt, und Fig. 3 zeigt eine Aufsicht auf die Unterseite des Tibiateils 1, welche in der normalen Gebrauchslage der Tibia zugewandt ist.

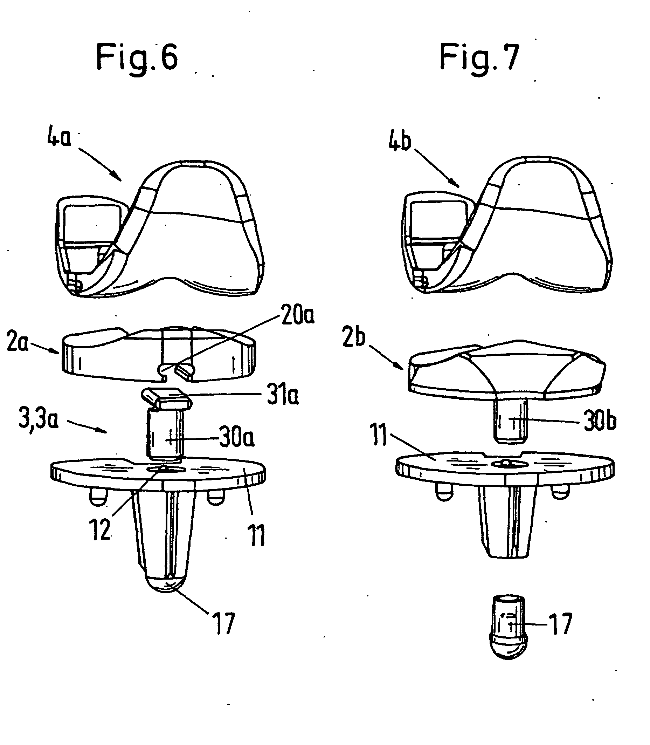

Das Tibiateil 1 hat eine Tibialagerfläche 11 zum Stützen eines Lagerkörpers 2 (siehe Fig. 6-9) und eine Bohrung 12 zur Aufnahme eines Führungselements 3 (siehe Fig. 6-9). Am posterioren Rand der Tibialagerfläche 11 ist eine Aussparung 13 für das hintere Kreuzband vorgesehen. Dadurch ist das Tibiateil 1 sowohl für solche Prothesentypen geeignet, bei denen das hintere Kreuzband erhalten bleibt, als auch für solche, bei den das hintere Kreuzband nicht mehr vorhanden ist bzw. nicht mehr funktionstüchtig ist. Das Tibiateil 1 weist ferner ein Ansatzstück 14 auf, welches sich im wesentlichen in Richtung der Längsachse erstreckt, und welches der Befestigung des Tibiateils 1 in der Tibia dient.

Dieses erste Ausführungsbeispiel ist insbesondere für solche Prothesentypen geeignet, bei denen der Lagerkörper 2 relativ zum Tibiateil 1 bewegbar gelagert ist. Die Bohrung 12 erstreckt sich von der Tibialagerfläche 11 durch das Ansatzstück 14 hindurch und ist so ausgestaltet, dass sie je nach Ausgestaltung des Führungselements 3 dieses entweder drehfest bezüglich des Tibiateils 1 oder drehbar bezüglich des Tibiateils 1 aufnehmen kann. Dazu umfasst die Bohrung 12 in diesem Ausführungsbeispiel einen oberen Bereich, der sich an die Tibialagerfläche 11 anschliesst, und der sich aus einem zylindrischen Teil 121 sowie einer sich seitlich daran anschliessenden nutförmigen Ausnehmung 122 zusammensetzt. Ist nun derjenige Bereich des Führungselements 3, der in die Bohrung 12 eingreift, im wesentlichen kreiszylindrisch oder konisch ausgebildet, so ist das Führungselement 3 bezüglich des Tibiateils 1 drehbar. Hat dagegen das Führungselement 3 in seinem mit der Bohrung 12 zusammenwirkenden Bereich zusätzlich einen Steg oder sonstigen Vorsprung, welcher in die nutförmige Ausnehmung 122 eingreift, so ist das Führungselement 3 drehfest bezüglich des Tibiateils 1 gelagert.

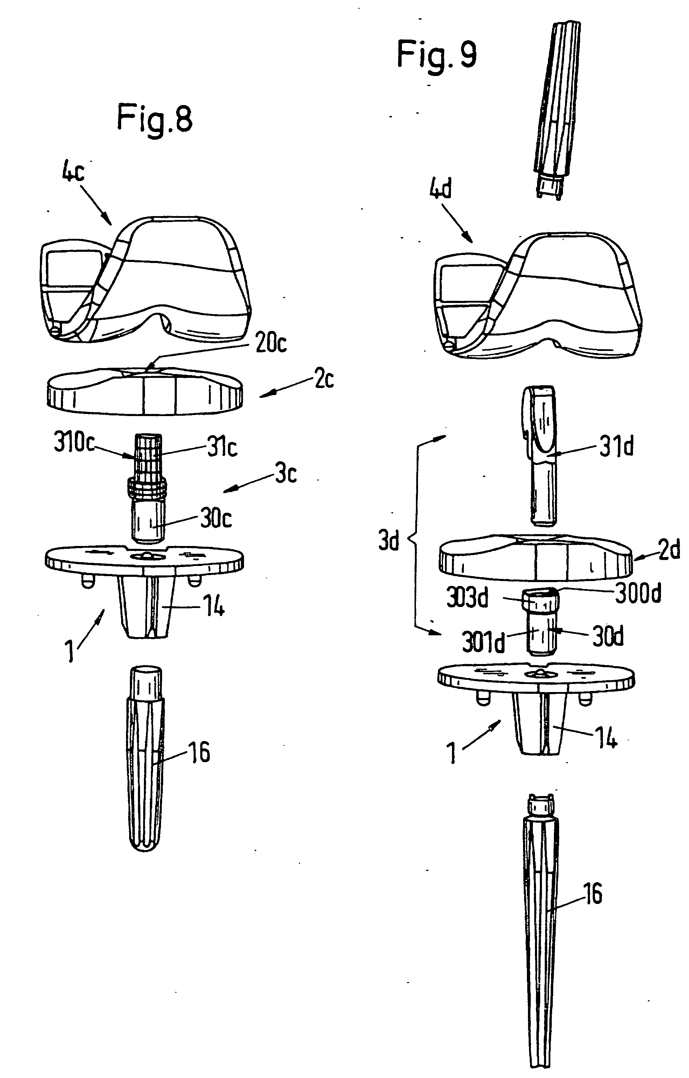

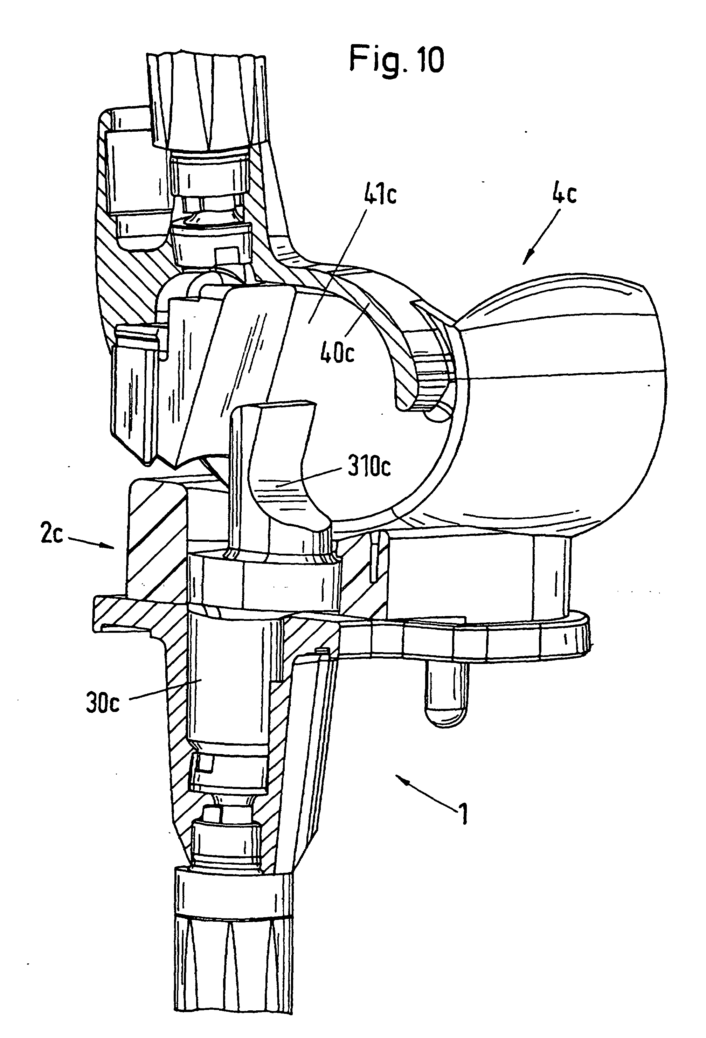

Nach unten hin wird der obere Bereich der Bohrung 12 durch einen wulstförmigen Absatz 123 begrenzt, der den Querschnitt der Bohrung 12 verengt. Darstellungsgemäss unterhalb des Absatzes 123 endet die Bohrung 12 mit einem zylindrischen unteren Bereich 124, welcher zur Aufnahme eines separaten Verankerungsschafts 16 (siehe Fig. 8 und Fig. 9) bzw. einer Verschlusskappe 17 (siehe Fig. 7) dient. Je nachdem, ob ein separater Verankerungsschaft 16 verwendet wird oder nicht, kann auch die Verschlusskappe 17 eingesetzt bleiben; sie ist zu diesem Zweck vorzugsweise aus einem mit dem zu verwendenden Knochenzement kompatiblen Material, z.B. aus Polymethylmethacrylat (PMMA) hergestellt. Um den separaten Verankerungsschaft 16 mit dem Ansatzstück 14 zu verbinden, wird das proximale Ende des Verankerungsschafts 16 in den unteren Bereich 124 der Bohrung 12 eingeführt. Dann wird von der Tibialagerfläche 11 aus eine Dehnschraube in die Bohrung 12 eingeführt, und in ein Gewinde geschraubt, welches im Ende des Verankerungsschafts 16 vorgesehen ist. Die Dehnschraube ist so bemessen, dass ihr Kopf nach dem Einschrauben auf dem Absatz 123 aufliegt, der als Anschlag dient. Auf diese Weise ist das Ansatzstück 14 fest mit dem separaten Verankerungsschaft 16 verbindbar.

Auf der Unterseite, mit welcher das Tibiateil 1 auf der Tibia aufliegt, sind mehrere Zementtaschen 15 zur Aufnahme von Knochenzement vorgesehen. In dieser Ausführungsform ist das Tibiateil 1 für solche Anwendungen geeignet, bei denen das Tibiateil 1 mittels Knochenzement an der Tibia fixiert wird. Das erste Ausführungsbeispiel des Tibiateils 1 kann aber auch in einfacher Weise für zementfreie Anwendungen ausgebildet werden. Dazu wird die Unterseite des Tibiateils mit einer Materialschicht versehen, welche das Einwachsen bzw. Festwachsen von Knochen fördert, beispielsweise mit einer Schicht aus porösem Titan. Zur Herstellung eines solchen Tibiateils für zementfreie Anwendungen können die Zementtaschen 15 eines Tibiateils für zementierte Anwendungen mit porösem Titan aufgefüllt werden, sodass die das Einwachsen bzw. Festwachsen des Knochens fördernde Materialschicht auf der Unterseite des Tibiateils 1 entsteht.

Abgesehen von dieser Materialschicht können die Tibiateile für zementfreie Anwendungen einerseits und für Anwendungen mit Zement andererseits identisch ausgebildet sein. Dies ist insbesondere herstellungstechnisch vorteilhaft, da für beide Arten von Anwendungen das gleiche Tibiatel produziert werden kann, wobei für die zementfreien Anwendungen lediglich ein weiterer Bearbeitungsschritt, nämlich das Füllen der Zementtaschen mit einem geeigneten Material, durchgeführt werden muss.

Auf der Unterseite des Tibiateils 1 können ein oder mehrere Stifte oder Zapfen 18 vorgesehen sein, um in einfacher Weise ein sicheres und richtiges Platzieren des Tibiateils 1 auf der Tibia zu gewährleisten, und um insbesondere bei der Implantation ein Verdrehen des Tibiateils 1 relativ zur Tibia zu verhindern.

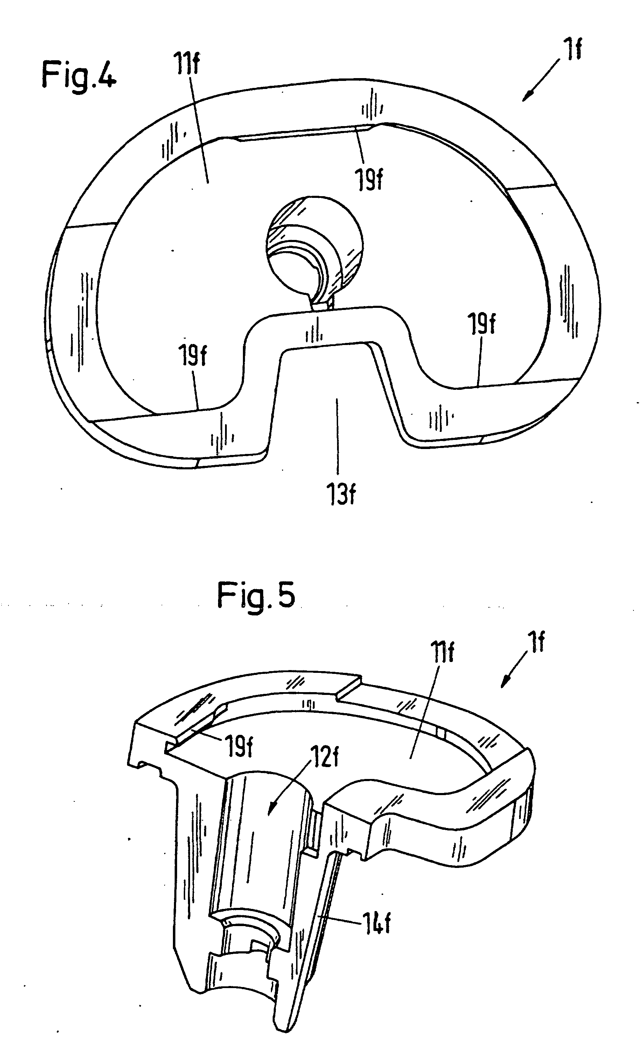

In den Figuren 4 und 5 ist ein zweites Ausführungsbeispiel eines erfindungsgemässen Tibiateils dargestellt, welches insbesondere für solche Prothesentypen geeignet ist, bei welchen der Lagerkörper relativ zum Tibiateil fixiert, also unbeweglich, ist. Die dem Ausführungsbeispiel gemäss Fig. 1-3 entsprechenden Teile sind jeweils mit dem Zusatz "f' (für "fixed") bezeichnet. Fig. 4 zeigt eine perspektivische Aufsicht auf das Tibiateil 1f, während Fig. 5 einen perspektivischen Längsschnitt durch das Tibiateil 1f zeigt.

Das zweite Ausführungsbeispiel des Tibiateils unterscheidet sich von dem ersten im wesentlichen nur dadurch, dass bei dem zweiten Ausführungsbeispiel Befestigungsmittel vorgesehen sind, um einen geeignet ausgebildeten Lagerkörper auf der Tibialagerfläche 11 f zu fixieren. Diese Befestigungsmittel können beispielsweise als Vorsprünge 19f realisiert sein, die mit entsprechenden Hinterschneidungen am Lagerkörper zusammenwirken, so wie dies bereits in der einleitend genannten Druckschrift EP-A-0,923,916 gezeigt ist (siehe insbesondere Fig. 5-10). Ansonsten gelten die Erläuterungen bezüglich des ersten Ausführungsbeispiels, welches anhand von Fig. 1-3 erläutert wurde, in sinngemäss gleicher Weise auch für das zweite Ausführungsbeispiel gemäss Fig. 4 und Fig. 5.

In den Fig. 6-9 sind vier verschiedene Typen von Kniegelenkprothesen gezeigt, welche alle das anhand der Fig. 1-3 erläuterte Ausführungsbeispiel des erfindungsgemässen Tibiateils 1 enthalten und aus einem Bausatz von verschiedenen Teilen zusammengestellt werden können, wie nachfolgend noch erläutert wird.

In Fig. 6 ist dabei ein Typ von Kniegelenkprothese gezeigt, der auch als Typ "CR" (Cruciate Retaining) bezeichnet wird. Bei diesem Typ von Kniegelenkprothese ist das hintere Kreuzband des Patienten funktionstüchtig und kann erhalten werden, ebenso sind die Seitenbänder noch funktionstüchtig und können erhalten werden. Das Führungselement 3 ist als Lenker 3a ausgebildet, dessen eines Ende als kreiszylindrischer Zapfen 30a ausgebildet ist, welcher nach dem Einbringen in die Bohrung 12 des Tibiateils 1 in der Bohrung 12 des Tibiateils 1 drehbar gelagert ist. Das andere stegartig ausgebildete Ende 31 a des Lenkers 3a greift in eine an der Unterseite des Lagerkörpers 2a vorgesehene Nut 20a ein. Weiterhin erkennt man in Fig. 6 noch ein Femurteil 4a, welches aber an sich bekannt ist.

Bei dem in Fig. 6 gezeigten Typ von Kniegelenkprothese ist der Lagerkörper 2a relativ zum Tibiateil 1 auf der Lagerfläche 11 des Tibiateils 1 drehbar und auch entlang des Stegs 31 a des Lenkers 3a in anteriorer bzw. posteriorer Richtung gleitend verschiebbar. Diese relativ hohe Freiheit der Kniegelenkprothese bedingt allerdings, wie bereits erwähnt, dass das hintere Kreuzband und die Seitenbänder des Patienten noch funktionstüchtig sind und erhalten bleiben können.

In Fig. 7 ist ein weiterer Typ von Kniegelenkprothese gezeigt, der auch als Typ "UCOR" (Ultra Congruent Only Rotating) bezeichnet wird. Dieser Typ von Kniegelenkprothese ist in erster Linie für solche Patienten bestimmt, bei denen das hintere Kreuzband nicht mehr vorhanden ist oder nicht mehr funktionstüchtig ist, bei denen jedoch die Seitenbänder noch funktionstüchtig sind. Es stellt also in gewisser Weise eine Alternative zu dem Typ "PS" dar, der anahnd von Fig. 8 noch eingehend erläutert werden wird.

Bei dem Typ "UCOR" gemäss Fig. 7 ist das Führungselement 3 als Zapfen 30b ausgebildet, welcher entweder direkt am Lagerkörper 2b angeformt ist (also Bestandteil des Lagerkörpers ist) oder aber an diesem fixiert ist. Der Zapfen 30b ist kreiszylindrisch ausgebildet, sodass er nach dem Einbringen in die Bohrung 12 des Tibiateils 1 in der Bohrung 12 des Tibiateils 1 drehbar gelagert ist. Somit ist der gesamte Lagerkörper 2b auf der Tibialagerfläche 11 relativ zum Tibiateil 1 drehbar, aber eben nur drehbar. Das Femurteil ist dadurch bestenfalls in sehr geringem Masse (nämlich in Stellungen, bei denen die Kondylen des Femurteils und die Lagerschalen des Lagerkörpers nicht genau kongruent sind) gegenüber dem Tibiateil 1 gleitend verschiebbar. Insgesamt weisen die Lagerschalen des Lagerkörpers 2b eine besonders hohe Kongruenz mit den Kondylen des Femurteils 4b auf, was einerseits zu einer guten Führung und aufgrund der grossen Kontaktfläche zwischen den Lagerschalen des Lagerkörpers 2b und den Kondylen des Femurteils 4b zu einer vergleichsweise geringen Flächenpressung führt. Darüberhinaus sind die Lagerschalen des Lagerkörpers 2b, die mit den Kondylen des Femurteils zusammenwirken, im posterioren Bereich auch etwas weiter nach oben gezogen, was die Luxationsgefahr verringert, die wegen des fehlenden hinteren Kreuzbandes erhöht ist.

In Fig. 8 ist ein weiterer Typ von Kniegelenkprothese gezeigt, der auch als Typ "PS" (Posterior Stabilized) bezeichnet wird. Dieser Typ von Kniegelenkprothese ist in erster Linie für solche Patienten bestimmt, bei denen das hintere Kreuzband nicht mehr vorhanden oder nicht mehr funktionstüchtig ist, hingegen sehr wohl noch die Seitenbänder (also ähnlich wie beim Typ "UCOR"). Sowohl beim Typ "PS" als auch bei dem später anhand von Fig. 9 noch zu erläuternden Typ von Kniegelenkprothese ist allerdings ein noch genauer zu erläuterndes Femurteil 4c Bestandteil der Prothese. Dieses noch zu erläuternde Femurteil ist grundsätzlich auch für die Typen von Kniegelenkprothese gemäss Fig. 6 und Fig. 7 (Typen "CR" und "UCOR") verwendbar, erfordert aber, dass etwas mehr Knochenmaterial vom Femurknochen entfernt wird, wie noch genauer erläutert werden wird.

Bei dem in Fig. 8 gezeigten Typ "PS" von Kniegelenkprothese, der nachstehend auch mit Hilfe von Fig. 10-13 erläutert wird, weist der Lagerkörper 2c ein durchgehendes Langloch 20c auf. Durch dieses Langloch 20c hindurch erstreckt sich das Führungselement 3c, dessen eines Ende als kreiszylindrischer Zapfen 30c ausgebildet ist. Dieser Zapfen 30c ist nach dem Einbringen in die Bohrung 12 im Tibiateil 1 drehbar gelagert. Das andere Ende 31 c des Führungselements 3c ragt zwischen die Kondylen des Femurteils 4c hinein und weist eine Führungsfläche 310c (siehe Fig. 10) auf, welche mit einem Stabilisierungselement im Femurteil 4c zusammenwirkt.

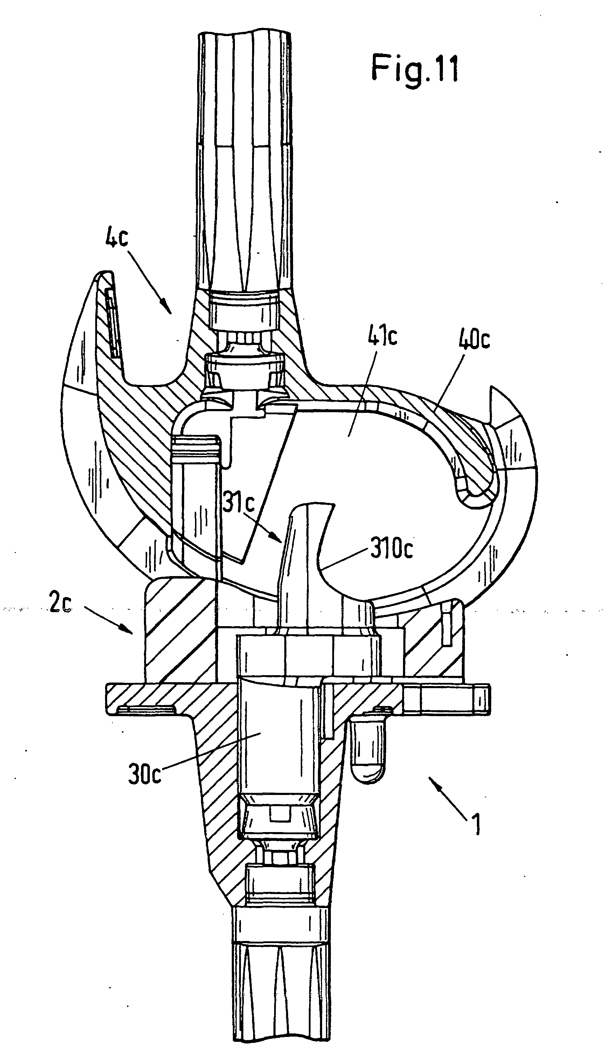

Dieses Stabilisierungselement ist hier ein wandartig ausgebildeter Verbindungssteg 40c, der zusammen mit den beiden Seitenwänden 41c, von denen in Fig. 10 nur eine dargestellt ist, einen Kasten definiert, in welchen das Ende 31 c des Führungselements 3c hineinragt. In Fig. 10 ist der Typ "PS" der Kniegelenkprothese in perspektivischer Darstellung im Zustand der Extension (Streckung) gezeigt, in Fig. 11 ist ebenfalls der Zustand der Extension dargestellt, jedoch im Längsschnitt und in Seitenansicht. Im Zustand der Extension befindet sich der Verbindungssteg 40c nicht in Eingriff mit der Führungsfläche 310c des Führungselements 3c.

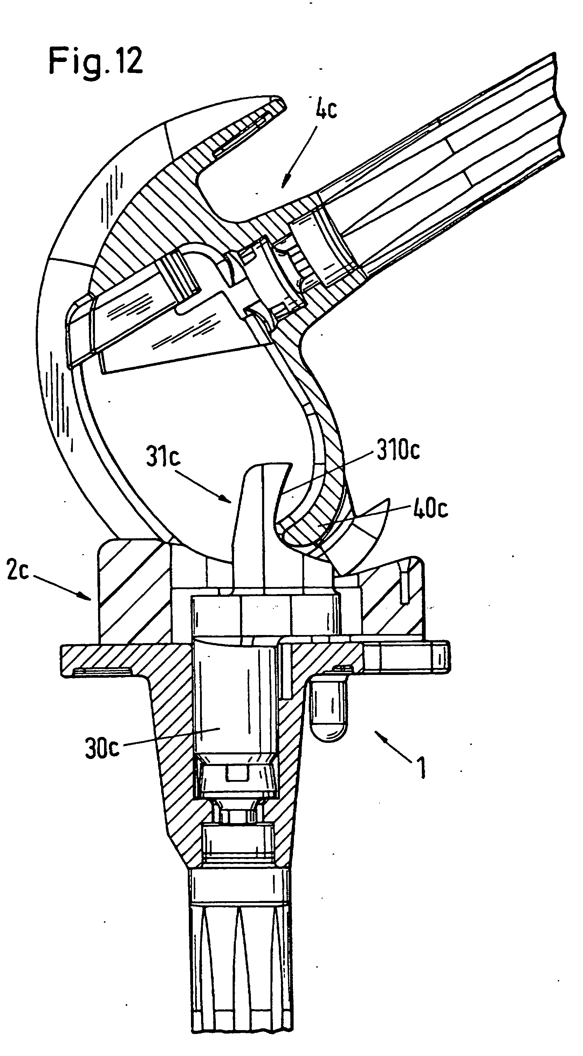

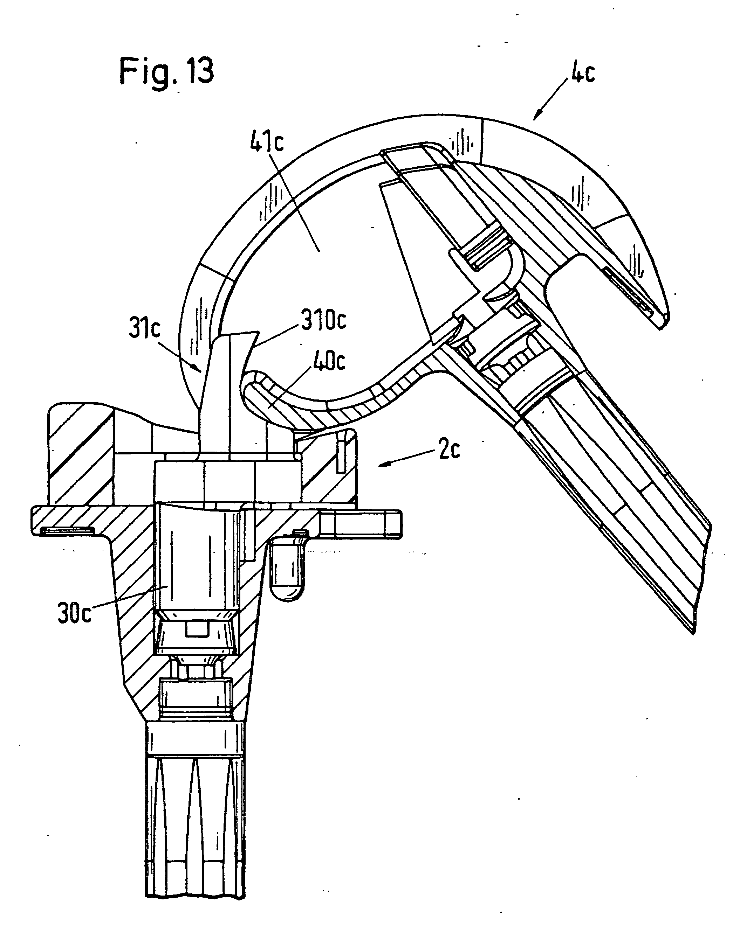

Erfolgt nun eine Flexion des Knies, so kommt ab einem bestimmten Flexionswinkel - siehe Fig. 12 - die Aussenwand des wandartig ausgebildeten Verbindungsstegs 40c mit der Führungsfläche 310c des Führungselements 3c in Eingriff. Das Femurteil 4c wird dadurch bei der weiteren Flexion davor bewahrt, wieder nach anterior zu gleiten, was ansonsten aufgrund des fehlenden hinteren Kreuzbands möglich wäre, es wird also in der posterioren Position stabilisiert. Diese Stabilisierung in der posterioren Position erfolgt dann bis hin zum Zustand der maximalen Flexion, der in Fig. 13 dargestellt ist, wobei hier aus zeichnerischen Gründen der Lagerkörper 2c nicht in der posterioren Position dargestellt ist, in welcher er sich in der Realität bei maximaler Flexion befindet.

In Fig. 9 ist ein weiterer Typ von Kniegelenkprothese gezeigt, der auch als Typ "SC" (Semi Constrained) bezeichnet wird. Dieser Typ von Kniegelenkprothese ist in erster Linie für solche Patienten bestimmt, bei denen weder das hintere Kreuzband noch die Seitenbänder vorhanden sind bzw. bei denen diese Bänder allesamt nicht mehr funktionstüchtig sind. Das Femurteil 4d dieses Typs stimmt bei diesem Typ von Kniegelenkprothese mit dem zuvor anhand von Fig. 8 und Fig. 10-13 beschriebenen Femurteil 4c völlig überein, für beide Typen von Kniegelenkprothese kann also ein und dasselbe Femurteil verwendet werden. Dieser Aspekt der Erfindung ist im Grunde eigenständig und unbhängig von der Verwendung eines universellen Tibiateils.

Das Führungselement 3d ist bei dem Typ "SC" gemäss Fig. 9 zweiteilig und weist ein Führungsstück 30d und ein Kopplungsstück 31d auf. Das Kopplungsstück 31d ist in eine Bohrung 300d des Führungsstücks einsetzbar und ist nach dem Einsetzen in diese Bohrung 300d in dieser Bohrung 300d drehbar gelagert. Das eine Ende des Führungsstücks 30d ist als Zapfen 301 d ausgebildet ist, welcher Zapfen nach dem Einbringen in die Bohrung 12 des Tibiateils 1 drehfest angeordnet ist. Während also das Kopplungsstück 31d in der Bohrung 300d des Führungsstück 30d drehbar gelagert ist, ist das Führungsstück 30d in der Bohrung 12 des Tibiateils 1 drehfest angeordnet.

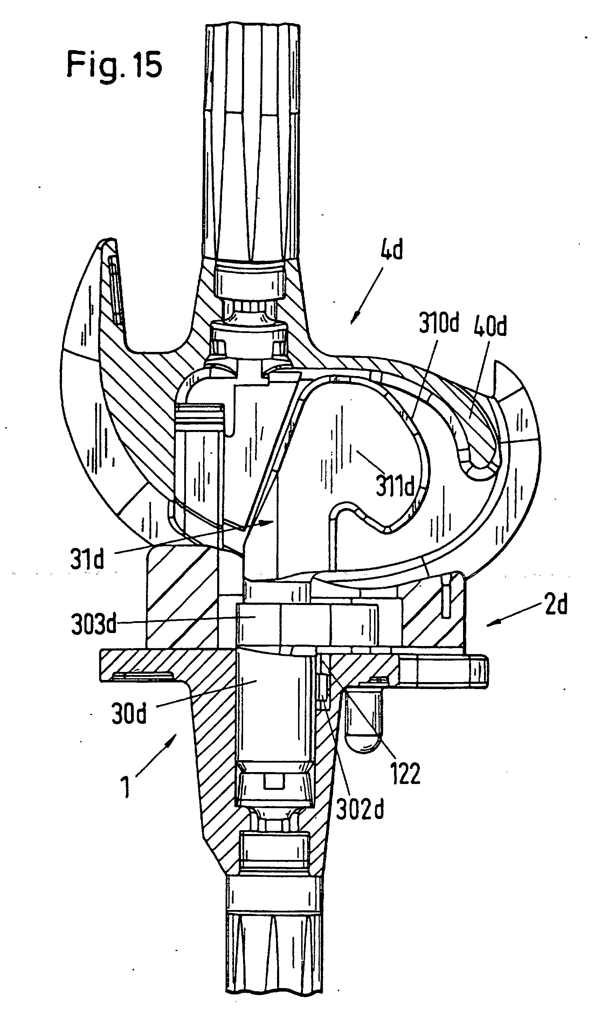

Zu diesem Zweck ist der Zapfen 301d des Führungsstücks 30d mit einem Vorsprung oder einer Finne 302d (Fig. 15) versehen, welche nach dem Einbringen des Zapfens 301d in die Bohrung 12 des Tibiateils in die nutförmige Ausnehmung 122 (siehe Fig. 2) der Bohrung 12 des Tibiateils 1 hineinreicht.

Der Lagerkörper 2d dieses Typs von Kniegelenkprothese entspricht dem Lagerkörper 2c des zuvor erläuterten Ausführungsbeispiels (siehe Fig. 8. bzw. Fig. 10-13). Das Führungsstück 30d weist ferner ein stegartig ausgebildetes Ende 303d auf, welches in das Langloch 20d des Lagerkörpers 2d eingreift. Da nun aber das Führungsstück 30d drehfest in der Bohrung 12 des Tibiateils 1 gelagert ist, ist auch der Lagerkörper 2 gegenüber dem Tibiateil 1 drehfest angeordnet.

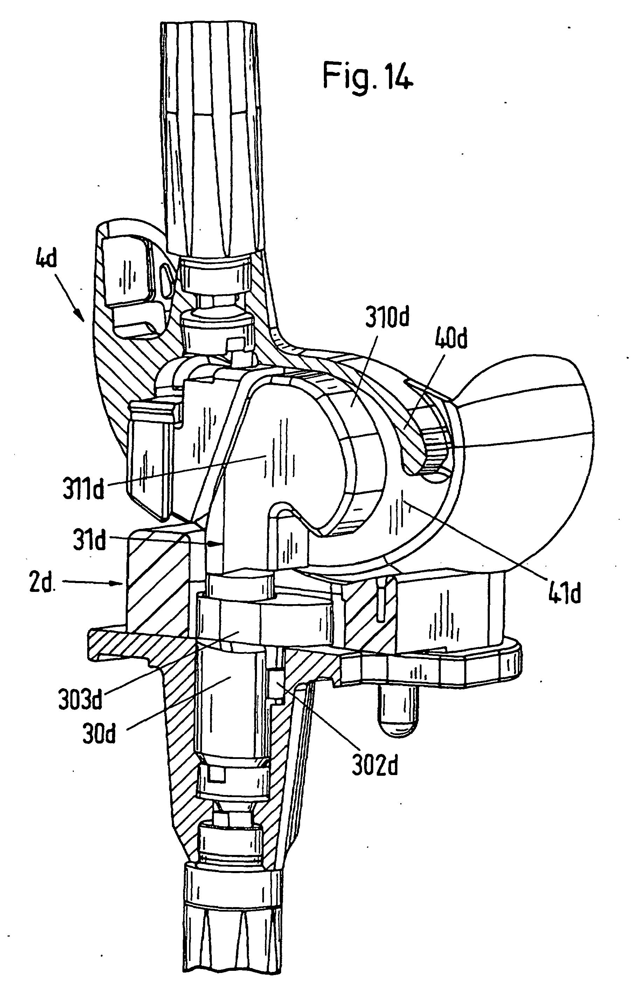

Der Typ "SC" der Prothese, der bereits in Fig. 9 in Explosionsdarstellung gezeigt ist, soll im Folgenden noch näher mit Hilfe der Fig. 14-17 erläutert werden. Fig. 14 zeigt dabei in perspektivischer Ansicht diesen Typ von Kniegelenkprothese im Zusammenbau im Zustand der Extension. Man erkennt den wandartigen Verbindungssteg 40d des Femurteils 4d, der zusammen mit den Seitenwänden 41d wieder einen Kasten bildet, völlig analog zu dem zuvor beschriebenen Ausführungsbeispiel (es ist ja das gleiche Femurteil).

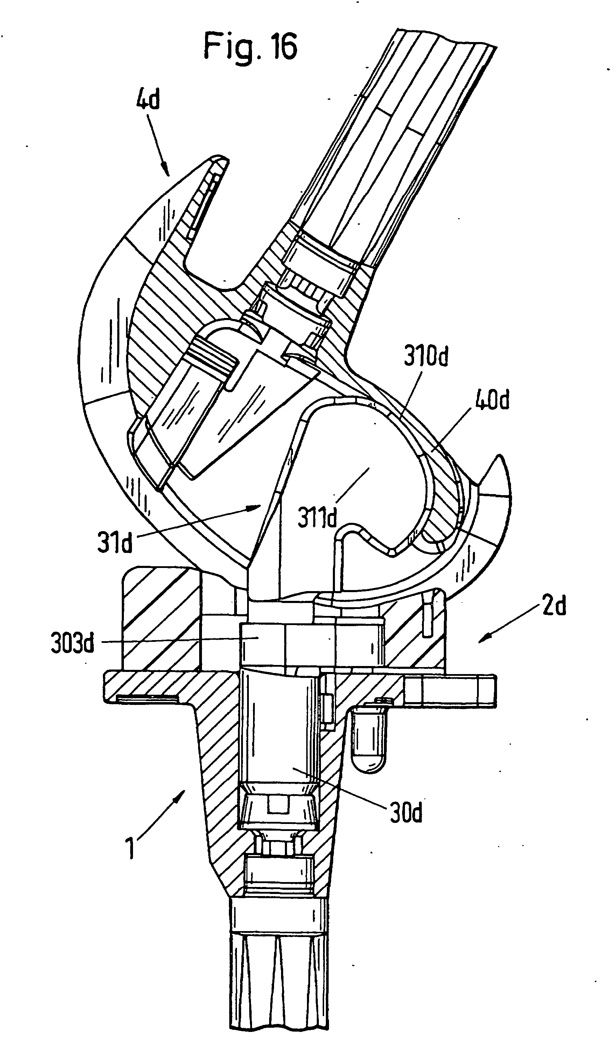

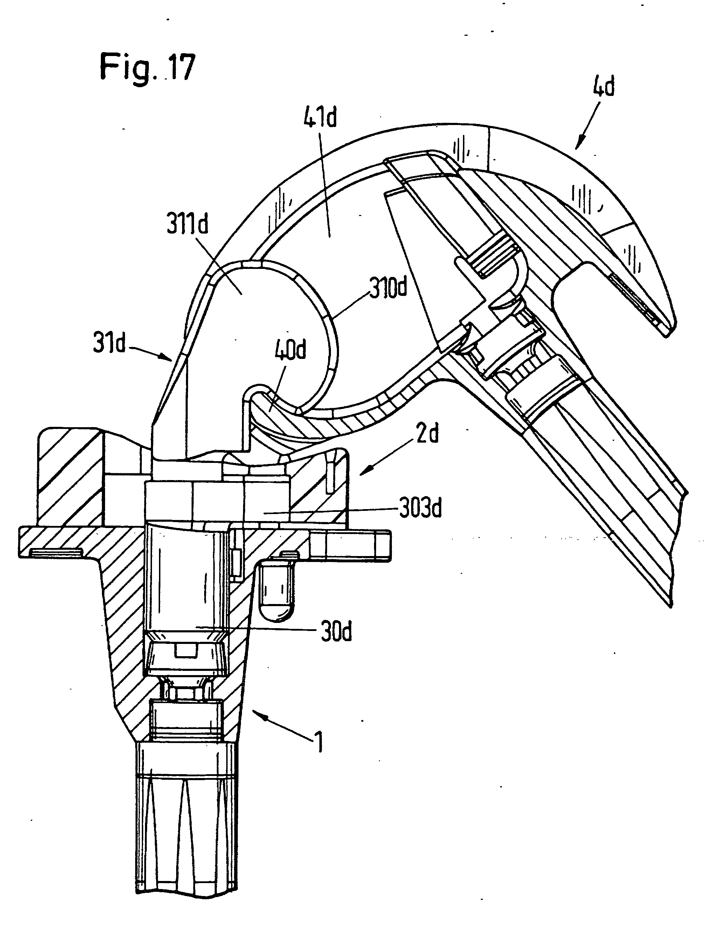

Ein wesentlicher Unterschied zum Typ "PS" besteht bei dem Typ "SC" darin, dass die Führungsfläche 310d des Führungsstücks 31d bei der Flexion nicht mit der Aussenwand, sondern mit der Innenwand des wandartigen Verbindungsstegs 40d in Eingriff kommt. Dies ist allerdings im Zustand der Extension, wie er in Fig. 14 und Fig. 15 gezeigt ist, noch nicht der Fall. Bei einer leichten Flexion kommt aber die Innenwand des wandartigen Verbindungsstegs 40d mit der Führungsfläche 310d des Führungsstücks 31d in Eingriff, wie dies in Fig. 16 gut zu erkennen ist, und bleibt dies auch bis zum Zustand der maximalen Flexion, der in Fig. 17 dargestellt ist. Dabei ist wieder anzumerken, dass sowohl in der Fig. 16 (leichte Flexion) als auch in der Fig. 17 (maximale Flexion) der Lagerkörper 2d in der Realität sich weiter posterior befinden würde als dies aus zeichnerischen Gründen in den betreffenden Figuren dargestellt ist.

Ein weiterer wesentlicher Unterschied ist der, dass das Kopplungsstück 31 d zwei seitliche Stabilisierungsflächen 311d aufweist, von denen in Fig. 14-17 jeweils nur eine zu erkennen ist. Diese Stabilisierungsflächen dienen der Varus/Valgus-Stabilisierung und kommen bei einer entsprechenden seitlichen Verkippung der Prothese mit der jeweiligen Seitenwand 41 d in Eingriff und verhindern so ein seitliches Verkippen des Femurteils.

Insgesamt weist also diese Prothese nicht mehr sehr viele Freiheitsgrade auf, die Bewegung ist grösstenteils geführt, allerdings lässt sie immer noch eine Verdrehung des Femurteils 4d relativ zum Lagerkörper 2d in gewissem Masse zu. Allerdings ist zu berücksichtigen, dass dieser Prothesentyp ja auch in erster Linie für solche Patienten bestimmt ist, bei denen sowohl das hintere Kreuzband als auch die Seitenbänder nicht mehr funktionstüchtig sind bzw. nicht mehr vorhanden sind. Die Artikulationsbewegung muss daher zwangsläufig einigermassen stark geführt sein.

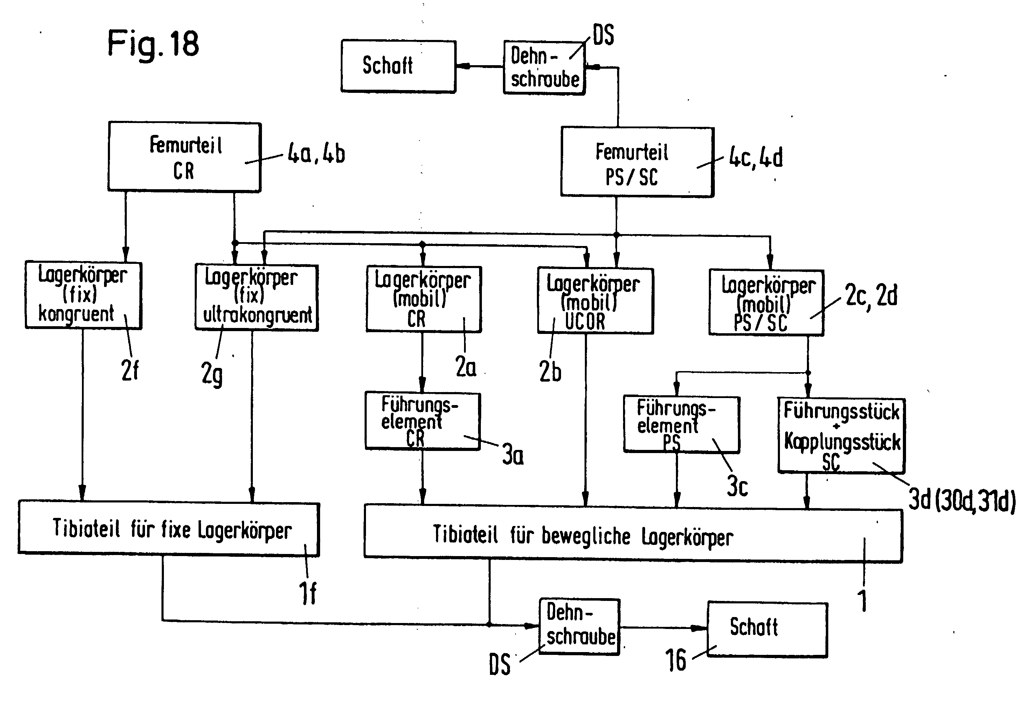

In Fig. 18 ist schliesslich eine Übersicht dargestellt, die die grosse Anzahl von Kombinationsmöglichkeiten zeigt, die dem Orthopäden praktisch noch intraoperativ zur Verfügung stehen. Dabei ist zu berücksichtigen, dass bei der Präparation der Tibia die Schnittführung bei der Resektion der Tibia praktisch unabhängig vom Typ der Kniegelenkprothese ist. Bei der Präparation des Femurs ist dies ähnlich: Die Schnittführung bei der Resektion ist praktisch für beide Arten von Femurteilen 4a,4b bzw. 4c,4d gleich, jedoch muss in dem Fall, in welchem ein Femurteil 4c,4d mit Kasten verwendet werden soll, dann anschliessend noch der Femur ausgespart werden für die Aufnahme des Kastens. Dies erleichtert dem Orthopäden die Präparation von Femur und Tibia und gibt ihm gleichzeitig die Möglichkeit, noch intraoperativ auf die anatomischen Gegebenheiten reagieren und den jeweils optimalen Prothesentyp implantieren zu können.

In der Übersicht in Fig. 18 erkennt man die zwei Typen von Tibiateilen, nämlich die Tibiateile 1 für verschiebbare und/oder drehbare Lagerkörper 2a,2b,2c,2d einerseits und die Tibiateile 1 a für fixierte Lagerkörper 2f und 2g andererseits. Die Lagerkörper 2g entsprechen den "UCOR"-Lagerkörpern für verschiebbare oder drehaber Lagerkörper, sind aber fest mit dem Tibiateil 1a verbunden.

Ferner erkennt man in Fig. 18 die verschiedenen Lagerkörper 2, nämlich die verschiebbaren und/oder drehbaren Lagerkörper 2a,2b,2c,2d einerseits und die fixierbaren Lagerkörper 2f und 2g andererseits. Darüberhinaus erkennt man bei den verschiebbaren und/oder drehbaren Lagerkörpern die verschiedenen Führungselemente 3a,3c,3d, im Falle des Führungselements 3d das Führungsstück 30d und das Kopplungsstück 31d.

Schliesslich erkennt man noch die beiden Typen von Femurteilen, nämlich die Typen 4a,4b ohne Kasten einerseits und die Typen 4c,4d mit Kasten andererseits. Je nach Zustand der Tibia kann mit dem jeweiligen Tibiateil 1,1a noch ein separater Schaft mit Hilfe einer Dehnschraube DS verbunden werden. Entsprechendes gilt für den Femur: Je nach Zustand kann mit dem jeweiligen Femurteil noch ein separater Schaft 5 verbunden werden, wobei es in beiden Fällen (Tibia und Femur) selbstverständlich ist, dass der Schaft in unterschiedlichen Grössen vorliegt.

Dies gilt natürlich auch für die übrigen Teile: Um einen vollständigen Bausatz vorliegen zu haben, müssen die einzelnen Teile vollständig in der jeweiligen Grösse vorhanden sein. In der Praxis ist jedoch die Anzahl der Teile, die tatsächlich im Operationssaal vorhanden sein müssen, sehr viel beschränkter, weil bei der präoperativen Planung ja die Grösse der Prothese bereits ermittelt wird. Es dürfte daher völlig ausreichen, wenn ausser der ermittelten Grösse jeweils noch die nächst grössere und die nächst kleinere Grösse der einzelnen Teile im Operationssaal vorhanden ist, um das volle Spektrum aller Kombinationsmöglichkeiten zur Verfügung zu haben. Nach der Operation braucht der Bausatz nur wieder aufgefüllt zu werden und bei der nächsten Implantation stehen wieder alle Möglichkeiten zur Verfügung.

Auch wenn klar ist, dass dann die Kombinationsmöglichkeiten maximal sind, wenn sämtliche in Fig. 18 vorgestellten Einzelteile bei einer Operation vorhanden sind, so ist es doch klar, dass auch solche Bausätze bereitgestellt werden können, die nur einen Teil sämtlicher Möglichkeiten abdecken. Beispielsweise kann der Orthopäde von vornherein festlegen, ob er einen verschiebbaren und/oder drehbaren Lagerkörper verwenden will, oder ob er zementiert oder zementfrei implantieren will, etc. . Infolgedessen wird der Bausatz dann jeweils nur eine Teilmenge des gesamten Bausatzes sein.

## Patentansprüche

1. Tibiateil (1,1f) für eine Kniegelenkprothese, mit einer Tibialagerfläche (11,11f) zum Stützen eines Lagerkörpers (2,2a,2b,2c,2d,2f,2g) und mit einer Bohrung (12,12f) zur Aufnahme eines Führungselements (3,3a,3c,3d), wobei in der Tibialagerfläche (11,11f) eine Aussparung (13,13f) für das hintere Kreuzband vorgesehen ist, und **dadurch gekennzeichnet, dass** die Bohrung (12,12f) derart ausgestaltet ist, dass sie je nach Ausgestaltung des Führungselements (3,3a,3c,3d) dieses entweder drehfest bezüglich des Tibiateils (1,1f) oder drehbar bezüglich des Tibiateils (1,1f) aufnehmen kann.

2. Tibiateil nach Anspruch 1, mit einem Ansatzstück (14,14f), welches der Befestigung des Tibiateils (1,1f) in der Tibia dient, wobei Verbindungsmittel (DS) vorgesehen sind, um das Ansatzstück (14,14f) fest mit einem separaten Verankerungsschaft (16) zu verbinden.

3. Tibiateil nach einem der vorangehenden Ansprüche, bei welchem Befestigungsmittel (19f) vorgesehen sind, um den Lagerkörper (2f,2g) auf der Tibialagerfläche (11f) zu fixieren.

4. Bausatz zum Zusammenstellen einer Kniegelenkprothese, mit mindestens einem Tibiateil (1,1f), das eine Tibialagerfläche (11,11f) aufweist, ferner mit mindestens einem Lagerkörper (2,2a,2b, 2c,2d,2f,2g), der auf der Tibialagerfläche (11,11f) lagerbar ist und der auf seiner der Tibialagerfläche (11,11f) abgewandten Seite Lagerschalen aufweist, sowie mit mindestens einem Femurteil (4,4a,4b,4c,4d), welches Kondylen zum Zusammenwirken mit den Lagerschalen des Lagerkörpers (2,2a,2b,2c,2d,2f,2g) aufweist, **dadurch gekennzeichnet, dass** das Tibiateil (1,1f) gemäss einem der vorangehenden Ansprüche ausgebildet ist.

5. Bausatz nach Anspruch 4, bei welchem mindestens ein Tibiateil (1f) und mindestens ein Lagerkörper (2f,2g) so ausgebildet sind, dass der Lagerkörper (2f,2g) auf der Tibialagerfläche (11f) fixierbar ist.

6. Bausatz nach einem der Ansprüche 4 oder 5, bei welchem mindestens ein Lagerkörper (2a) gleitend verschiebbar auf der Tibialagerfläche (11) lagerbar ist, und bei welchem mindestens ein Führungselement (3) vorgesehen ist, das einen Lenker (3a) umfasst, dessen eines Ende (30a) so ausgebildet ist, dass es drehbar in der Bohrung (12) des Tibiateils (1) lagerbar ist, und der einen Führungsabschnitt (31 a) aufweist, welcher so ausgebildet ist und mit dem Lagerkörper (2a) zusammenwirkt, dass der Lagerkörper (2a) relativ zur Tibialagerfläche (11) in anteriorer/posteriorer Richtung verschiebbar ist.

7. Bausatz nach einem der Ansprüche 4-6, bei welchem mindestens ein Lagerkörper (2b) auf seiner der Tibialagerfläche (11) zugewandten Seite ein angeformtes oder fixiertes Führungselement (30b) aufweist, mittels welchem der Lagerkörper (2b) drehbar in der Bohrung (12) des Tibiateils (1) lagerbar ist.

8. Bausatz nach einem der Ansprüche 4-7, bei welchem mindestens ein Lagerkörper (2c) ein axial durchgangiges Langloch (20c) aufweist, und bei welchem mindestens ein Führungselement (3c) vorgesehen ist, welches sich durch das Langloch (20c) des Lagerkörpers (2c) hindurch erstreckt, wobei das eine Ende des Führungselements als Zapfen (30c) ausgebildet ist, der sich in die Bohrung (12) im Tibiateil (1) hinein erstreckt und dort drehbar gelagert ist, und das andere Ende (31c) zwischen die Kondylen des Femurteils (4c) hineinragt und eine Führungsfläche (310c) zum Zusammenwirken mit einem im Femurteil vorgesehenen Stabilisierungselement (40c) aufweist.

9. Bausatz nach einem der Ansprüche 4-8, bei welchem mindestens ein Führungselement (3d) so ausgebildet ist, dass es ein Führungsstück (30d) und ein Kopplungsstück (31d) aufweist, wobei das Führungsstück (30d) so ausgebildet ist, dass es sowohl drehfest bezüglich des Tibiateils (1) in der Bohrung (12) des Tibiateils (1) angeordnet werden kann, als auch drehfest mit dem Lagerkörper (2d) verbindbar ist, und wobei das Kopplungsstück (31 d) drehbar in dem Führungsstück (30d) gelagert ist und eine Stabilisierungsstück aufweist, welches zwischen die Kondylen des Femurteils (4d) reicht und mit einer Führungsfläche (310d) versehen ist, um dort mit einem am Femurteil (4d) vorgesehenen Stabilisierungselement (40d) zusammenzuwirken.

10. Bausatz nach einem der Ansprüche 8 oder 9, bei welchem das Stabilisierungselement (4c,4d) am Femurteil einen wandartig ausgebildeten Verbindungssteg (40c,40d) umfasst, der zusammen mit zwei Seitenwänden (41c,41d) einen Kasten bildet, welcher zwischen den beiden Kondylen angeordnet ist und welcher mit der Führungsfläche (310c,310d) des Führungselements (3c) bzw. mit dem Stabilisierungsstück des Kopplungsstücks (31 d) des Führungselements (3d) zusammenwirkt.

## Claims

1. Tibia part (1, 1f) for a knee joint prosthesis, having a tibia bearing surface (11, 11f) for the support of a bearing body (2, 2a, 2b, 2c, 2d, 2f, 2g) and having a bore (12, 12f) for the reception of a guide element (3, 3a, 3c, 3d), wherein a cut-out (13, 13f) for the rear cruciate ligament is provided in the tibia bearing surface (11, 11f), and **characterized in that** the bore (12, 12f) is formed such that, depending on the design of the guide element (3, 3a, 3c, 3d), it can receive the latter either rotationally fixed relative to the tibia part (1, 1f) or rotatabe relative to the tibia part (1, 1f).

2. Tibia part in accordance with claim 1, comprising a projection (14, 14f) which serves for securing the tibia part (1, 1f) in the tibia, with connection means (DS) being provided in order to connect the projection (14, 14f) firmly to a separate anchoring shaft (16).

3. Tibia part in accordance with any one of the preceding claims, in which securing means (19f) are provided in order to fix the bearing body (2f, 2g) on the tibia bearing surface (11f).

4. Kit for putting together a knee joint prosthesis, having at least one tibia part (1, 1f) which has a tibia bearing surface (11, 11f), further having at least one bearing body (2, 2a, 2b, 2c, 2d, 2f, 2g) which can be journalled on the tibia bearing surface (11, 11f) and which has bearing shells on its side remote from the tibia bearing surface (11, 11f), as well as having at least one femur part (4, 4a, 4b, 4c, 4d) which has condyles for cooperation with the bearing shells of the bearing body (2, 2a, 2b, 2c, 2d, 2f, 2g), **characterized in that** the tibia part (1, 1f) is formed in accordance with any one of the preceding claims.

5. Kit in accordance with claim 4, in which at least one tibia part (1f) and at least one bearing body (2f, 2g) are formed such that the bearing body (2f, 2g) can be fixed at the tibia bearing surface (11f).

6. Kit in accordance with claim 4 or claim 5, in which at least one bearing body (2a) can be slidingly displaceably journalled on the tibia bearing surface (11) and in which at least one guide element (3) is provided which comprises a link (3a), the one end (30a) of which is formed such that it can be rotatably supported in the bore (12) of the tibia part (1) and which has a guide section (31a) which is formed and which cooperates with the bearing body (2a) such that the bearing body (2a) is displaceable relative to the tibia bearing surface (11) in the anterior/posterior direction.

7. Kit in accordance with any one of the claims 4 - 6, in which at least one bearing body (2b) has, at its side facing the tibia bearing surface (11), a molded or fixed guide element (30b) by means of which the bearing body (2b) can be rotatably supported in the bore (12) of the tibia part (1),

8. Kit in accordance with any one of the claims 4 - 7, in which at least one bearing body (2c) has an axial through-going elongate hole (20c) and in which at least one guide element (3c) is provided which passes through the elongate hole (20c) of the bearing body (2c), with the one end of the guide element being formed as a spigot (30c) which extends into the bore (12) in the tibia part (1) and is rotatably supported there, and with the other end (31c) protruding between the condyles of the femur part (4c) and having a guide surface (310c) for cooperating with a stabilizing element (40c) which is provided in the femur part.

9. Kit in accordance with any one of the claims 4 - 8, in which at least one guide element (3d) is formed such that it has a guide piece (30d) and a coupling piece (31d), with the guide piece (30d) being formed such that it can be both rotationally fixedly arranged relative to the tibia part (1) in the bore (12) of the tibia part (1) and rotationally fixedly connected to the bearing body (2d), and with the coupling piece (31d) being rotatably supported in the guide piece (30d) and having a stabilizing piece which extends between the condyles of the femur part (4d) and is provided with a guide surface (310d) in order to cooperate there with a stabilizing element (40d) provided at the femur part (4d).

10. Kit in accordance with any one of the claims 8 or 9, in which the stabilizing element (4c, 4d) at the femur part comprises a connection web (40c, 40d) which is formed in the manner of a wall and which, together with two side walls (41c, 41d, forms a box which is arranged between the two condyles and which cooperates with the guide surface (310c, 310d) of the guide element (3c) or with the stabilizing piece of the coupling piece (31d) of the guide element (3d).

## Revendications

1. Pièce tibiale (1, 1f) pour une prothèse de l'articulation du genou, avec une surface de positionnement tibiale (11, 11f) pour soutenir un plateau (2, 2a, 2b, 2c, 2d, 2f, 2g) et avec un alésage (12, 12f) pour recevoir un élément de guidage (3, 3a, 3c, 3d), un évidement (13, 13f) étant prévu dans la surface de positionnement tibiale (11, 11f) pour le ligament croisé arrière et **caractérisée en ce que** l'alésage (12, 12f) est formé de manière à pouvoir selon la forme de l'élément de guidage (3, 3a, 3c, 3d), recevoir celui-ci sans rotation par rapport à la pièce tibiale (1, 1f) ou par rotation par rapport à la pièce tibiale (1, 1f).

2. Pièce tibiale selon la revendication 1, avec un embout (14, 14f) qui sert à fixer la pièce tibiale (1, 1f) dans le tibia, un moyen de liaison (DS) étant prévu pour fixer l'embout (14, 14f) avec une tige d'ancrage (16) séparée.

3. Pièce tibiale selon l'une des revendications précédentes, dans laquelle des moyens de fixation (19f) sont prévus pour fixer le plateau (2f, 2g) sur la surface de positionnement tibiale (11f).

4. Ensemble modulaire pour la mise en place d'une prothèse de l'articulation du genou, avec au moins une pièce tibiale (1, 1f) qui présente une surface de positionnement tibiale (11, 11f), avec en outre au moins un plateau (2, 2a, 2b, 2c, 2d, 2f, 2g), pouvant être positionné sur la surface de positionnement tibiale (11, 11f) et présentant des coques sur sa surface opposée à la surface de positionnement tibiale (11, 11f), ainsi qu'avec au moins une pièce fémorale (4, 4a, 4b, 4c, 4d) présentant des condyles pour agir conjointement avec les coques du plateau (2, 2a, 2b, 2c, 2d, 2f, 2g), **caractérisé en ce que** la pièce tibiale (1, 1f) est formée selon l'une des revendications précédentes.

5. Ensemble modulaire selon la revendication 4, dans lequel au moins une pièce tibiale (1f) et au moins un plateau (2f, 2g) sont formés de façon à ce que le plateau (2f, 2g) puisse être fixé sur la surface de positionnement tibiale (11f).

6. Ensemble modulaire selon l'une des revendications 4 ou 5, dans lequel au moins un plateau (2a) peut être positionné déplaçable par glissement sur la surface de positionnement tibiale (11) et dans lequel au moins un élément de guidage (3) est prévu, comprenant un bras oscillant (3a) dont une extrémité (30a) est formée de façon à être positionnable par rotation dans l'alésage (12) de la pièce tibiale (1) et qui présente une section de guidage (31a) formée et agissant avec le plateau (2a) de manière à ce que le plateau (2a) soit déplaçable dans une direction antérieure/postérieure à la surface de positionnement tibiale.

7. Ensemble modulaire selon l'une des revendications 4 à 6, dans lequel au moins un plateau (2b) présente un élément de guidage (30b) fixé ou formé sur sa surface en contact avec la surface de positionnement tibiale (11), au moyen duquel le plateau (2b) est positionnable par rotation dans l'alésage (12) de la pièce tibiale (1).

8. Ensemble modulaire selon l'une des revendications 4 à 7, dans lequel au moins un plateau (2c) présente un alésage longitudinal (20c) axial et dans lequel au moins un élément de guidage (3c) est prévu, passant à travers l'alésage longitudinal (20c) du plateau (2c), l'une extrémité de l'élément de guidage ayant la forme d'une cheville (30c) s'étendant à travers l'alésage (12) de la pièce tibiale (1) et étant positionnée par rotation à cet endroit, et l'autre extrémité (31c) faisant saillie entre les condyles de la pièce fémorale (4c) et présentant une surface de guidage (310c) pour agir conjointement avec un élément de stabilisation (40c) prévu dans la pièce fémorale.

9. Ensemble modulaire selon l'une des revendications 4 à 8, dans lequel au moins un élément de guidage (3d) est formé de manière à présenter une pièce de guidage (30d) et une pièce d'assemblage (31d), la pièce de guidage (30d) étant formée de manière à pouvoir aussi bien être placée sans rotation par rapport à la pièce tibiale (1) dans l'alésage (12) de la pièce tibiale (1), que reliable sans rotation au plateau (2d), et la pièce d'assemblage (31d) étant positionnée par rotation dans la pièce de guidage (30d) et présentant une pièce de stabilisation, qui pénètre entre les condyles de la pièce fémorale (4d) et est munie d'une surface de guidage (310d) pour agir conjointement à cet endroit avec un élément de stabilisation (40d) prévu sur la pièce fémorale (4d).

10. Ensemble modulaire selon l'une des revendications 8 ou 9, dans lequel l'élément de stabilisation (4c, 4d) comprend une ailette de liaison (40c, 40d) formant paroi sur la pièce fémorale, qui forme avec deux parois latérales (41c, 41d) un logement placé entre les deux condyles et agissant conjointement avec la surface de guidage (310c, 310d) de l'élément de guidage (3c), ou avec la pièce de stabilisation de la pièce d'assemblage (31d) de l'élément de guidage (3d).
